# EUROPÄISCHE PATENTSCHRIFT

(11) **EP 2 513 305 B1**
(45) Veröffentlichungstag und Bekanntmachung des Hinweises auf die Patenterteilung: **23.07.2014**
(21) Anmeldenummer: 10798773.7
(22) Anmeldetag: 17.12.2010
(51) Int. Cl.: C12N 15/10, C07K 14/37, C12N 15/80, C12P 21/02, C12N 15/81

(54) **SEKRETIONSSYSTEM AUF BASIS EINER HYDROPHOBIN-SIGNALSEQUENZ AUS TRICHODERMA REESEI**
SECRETION SYSTEM ON THE BASIS OF A HYDROPHOBIN SIGNAL SEQUENCE FROM TRICHODERMA REESEI
SYSTÈME DE SÉCRÉTION À BASE D'UNE SÉQUENCE SIGNAL D'HYDROPHOBINE DE TRICHODERMA REESEI

(30) Priorität: 18.12.2009 DE 102009055015
(43) Veröffentlichungstag der Anmeldung: 24.10.2012
(73) Patentinhaber: Technische Universität Dresden, 01069 Dresden (DE)
(72) Erfinder: KOTTMEIER, Kirsten, 01187 Dresden (DE); OSTERMANN, Kai, 01187 Dresden (DE); RÖDEL, Gerhard, 85757 Karlsfeld (DE)
(74) Vertreter: Kailuweit & Uhlemann Patentanwälte
(86) Internationale Anmeldenummer: PCT/EP2010/070022
(87) Internationale Veröffentlichungsnummer: WO 2011/073367

(56) Entgegenhaltungen:
- WO-A1-01/79558
- WO-A2-2008/073914
- US-A1- 2004 002 136
- US-A1- 2006 040 349
- NAKARI-SETALA T ET AL: "GENETIC AND BIOCHEMICAL CHARACTERIZATION OF THE TRICHODERMA RESEI HYDROPHOBIN HFBI", EUROPEAN JOURNAL OF BIOCHEMISTRY, BLACKWELL PUBLISHING, BERLIN, DE, Bd. 235, 1. Januar 1996 (1996-01-01), Seiten 248-255, XP002949769, ISSN: 0014-2956, DOI: DOI:10.1111/J.1432-1033.1996.00248.X
- NAKARI-SETALA T ET AL: "DIFFERENTIAL EXPRESSION OF THE VEGETATIVE AND SPORE-BOUND HYDROPHOBINS OF TRICHODERMA REESEI CLONING AND CHARACTERIZATION OF THE HFB2 GENE", EUROPEAN JOURNAL OF BIOCHEMISTRY, PUBLISHED BY SPRINGER-VERLAG ON BEHALF OF THE FEDERATION OF EUROPEAN BIOCHEMICAL SOCIETIES, vol. 248, 1 January 1997 (1997-01-01), pages 415-423, XP002949768, ISSN: 0014-2956, DOI: 10.1111/J.1432-1033.1997.00415.X

### Sequenzprotokoll

<110> Technische Universität Dresden
<120> Sekretionssystem auf Basis einer Hydrophobin-Signalsequenz aus Trichoderma reesei
<130> 00017P0145DEWO
<150> DE 10 2009 055 015
   <151> 2009-12-18
<160> 28
<170> PatentIn version 3.5
<210> 1
   <211> 10
   <212> PRT
   <213> Trichoderma reesei
<400> 1
   Met Lys Phe Phe Ala Ile Ala Ala Leu Phe
   1               5                   10
<210> 2
   <211> 11
   <212> PRT
   <213> Trichoderma reesei
<400> 2
   Met Lys Phe Phe Ala Ile Ala Ala Leu Phe Ala
   1               5                   10
<210> 3
   <211> 12
   <212> PRT
   <213> Trichoderma reesei
<400> 3
   Met Lys Phe Phe Ala Ile Ala Ala Leu Phe Ala Ala
   1               5                   10
<210> 4
   <211> 13
   <212> PRT
   <213> Trichoderma reesei
<400> 4
   Met Lys Phe Phe Ala Ile Ala Ala Leu Phe Ala Ala Ala
   1               5                   10
<210> 5
   <211> 14
   <212> PRT
   <213> Trichoderma reesei
<400> 5
   Met Lys Phe Phe Ala Ile Ala Ala Leu Phe Ala Ala Ala Ala
   1               5                   10
<210> 6
   <211> 15
   <212> PRT
   <213> Trichoderma reesei
<400> 6
   Met Lys Phe Phe Ala Ile Ala Ala Leu Phe Ala Ala Ala Ala Val
   1               5                   10                  15
<210> 7
   <211> 16
   <212> PRT
   <213> Trichoderma reesei
<400> 7
   Met Lys Phe Phe Ala Ile Ala Ala Leu Phe Ala Ala Ala Ala Val Ala
   1 5 10 15
<210> 8
   <211> 17
   <212> PRT
   <213> Trichoderma reesei
<400> 8
   Met Lys Phe Phe Ala Ile Ala Ala Leu Phe Ala Ala Ala Ala Val Ala
   1 5 10 15
   Gln
<210> 9
   <211> 18
   <212> PRT
   <213> Trichoderma reesei
<400> 9
   Met Lys Phe Phe Ala Ile Ala Ala Leu Phe Ala Ala Ala Ala Val Ala
   1 5 10 15
   Gln Pro
<210> 10
   <211> 19
   <212> PRT
   <213> Trichoderma reesei
<400> 10
   Met Lys Phe Phe Ala Ile Ala Ala Leu Phe Ala Ala Ala Ala Val Ala
   1 5 10 15
   Gln Pro Leu
<210> 11
   <211> 20
   <212> PRT
   <213> Trichoderma reesei
<400> 11
   Met Lys Phe Phe Ala Ile Ala Ala Leu Phe Ala Ala Ala Ala Val Ala
   1 5 10 15
   Gln Pro Leu Glu
   20
<210> 12
   <211> 21
   <212> PRT
   <213> Trichoderma reesei
<400> 12
   Met Lys Phe Phe Ala Ile Ala Ala Leu Phe Ala Ala Ala Ala Val Ala
   1 5 10 15
   Gln Pro Leu Glu Asp
   20
<210> 13
   <211> 22
   <212> PRT
   <213> Trichoderma reesei
<400> 13
   Met Lys Phe Phe Ala Ile Ala Ala Leu Phe Ala Ala Ala Ala Val Ala
   1 5 10 15
   Gln Pro Leu Glu Asp Arg
   20
<210> 14
   <211> 15
   <212> PRT
   <213> Trichoderma reesei
<400> 14
   Met Gln Phe Phe Ala Val Ala Leu Phe Ala Thr Ser Ala Leu Ala
   1 5 10 15
<211> 30
   <212> DNA
   <213> Trichoderma reesei
<400> 15
   atgaagttct tcgccatcgc cgctctcttt 30
<210> 16
   <211> 33
   <212> DNA
   <213> Trichoderma reesei
<400> 16
   atgaagttct tcgccatcgc cgctctcttt gcc 33
<210> 17
   <211> 36
   <212> DNA
   <213> Trichoderma reesei
<400> 17
   atgaagttct tcgccatcgc cgctctcttt gccgcc 36
<210> 18
   <211> 39
   <212> DNA
   <213> Trichoderma reesei
<400> 18
   atgaagttct tcgccatcgc cgctctcttt gccgccgct 39
<210> 19
   <211> 42
   <212> DNA
   <213> Trichoderma reesei
<400> 19
   atgaagttct tcgccatcgc cgctctcttt gccgccgctg cc 42
<210> 20
   <211> 45
   <212> DNA
   <213> Trichoderma reesei
<400> 20
   atgaagttct tcgccatcgc cgctctcttt gccgccgctg ccgtt 45
<210> 21
   <211> 48
   <212> DNA
   <213> Trichoderma reesei
<400> 21
   atgaagttct tcgccatcgc cgctctcttt gccgccgctg ccgttgcc 48
<210> 22
   <211> 51
   <212> DNA
   <213> Trichoderma reesei
<400> 22
   atgaagttct tcgccatcgc cgctctcttt gccgccgctg ccgttgccca g 51
<210> 23
   <211> 54
   <212> DNA
   <213> Trichoderma reesei
<400> 23
   atgaagttct tcgccatcgc cgctctcttt gccgccgctg ccgttgccca gcct 54
<210> 24
   <211> 57
   <212> DNA
   <213> Trichoderma reesei
<400> 24
   atgaagttct tcgccatcgc cgctctcttt gccgccgctg ccgttgccca gcctctc 57
<210> 25
   <211> 60
   <212> DNA
   <213> Trichoderma reesei
<400> 25
   atgaagttct tcgccatcgc cgctctcttt gccgccgctg ccgttgccca gcctctcgag 60
<210> 26
   <211> 63
   <212> DNA
   <213> Trichoderma reesei
<400> 26
   atgaagttct tcgccatcgc cgctctcttt gccgccgctg ccgttgccca gcctctcgag 60
gac 63
<210> 27
   <211> 66
   <212> DNA
   <213> Trichoderma reesei
<400> 27
   atgaagttct tcgccatcgc cgctctcttt gccgccgctg ccgttgccca gcctctcgag 60
gaccgc 66
<210> 28
   <211> 45
   <212> DNA
   <213> Trichoderma reesei
<400> 28
   atgcagttct tcgccgtcgc cctcttcgcc accagcgccc tggct 45

## Beschreibung

Die vorliegende Erfindung betrifft ein Sekretionssystem auf Basis einer Signalsequenz aus einem Hydrophobin-Protein aus *Trichoderma reesei,* das die Sekretion von rekombinanten Proteinen aus eukaryotischen Zellen, insbesondere Hefezellen, vermittelt. Das Sekretionssystem eignet sich besonders für die Anwendung in der Molekularbiologie und in der Biotechnologie.

Bei der Herstellung rekombinanter Proteine durch eukaryotische Zellen, wie z. B. Hefezellen wie *Pichia pastoris,* ist es von Vorteil, wenn das Protein aus der Zelle in das Medium sezerniert wird. Dadurch wird die Aufreinigung des Proteins erheblich erleichtert, da aufwändige Bearbeitungsschritte, wie z. B. der Aufschluss der Zellen, entfallen. Da Hefen oft nur geringe Mengen an eigenem Protein ausschleusen, ist das sezernierte rekombinante Protein außerdem im Kulturüberstand in relativer Reinheit zu finden. Dies gestattet es, das Protein mit einfachen Verfahren schnell und effizient zu reinigen.

Damit ein rekombinant zu exprimierendes Protein (Zielprotein) erfolgreich aus der Zelle ausgeschleust werden kann, muss es eine Signalsequenz enthalten, durch welche das betreffende Protein in das Endoplasmatische Retikulum als Eintrittsstelle für den sekretorischen Pathway geleitet wird. Sofern das Zielprotein kein authentisches Sekretionssignal besitzt, wird üblicherweise das Zielprotein mittels molekularbiologischer Methoden mit einer bekannten Signalsequenz fusioniert.

Ein Peptid, welches einen effizienten Transport von rekombinanten Proteinen durch die Cytoplasmamembran ermöglicht, ist daher ein nützliches Werkzeug in der Biotechnologie. Im Stand der Technik wurde bisher hauptsächlich die Signalsequenz des Pheromons α-Faktor aus *Saccharomyces cerevisiae* als Sekretionssignal verwendet. Die Signalsequenz der sauren Phosphatase aus *Pichia pastoris* gehört ebenfalls zu den kommerziell erhältlichen Sekretionssignalen (Cereghino JL, Cregg JM. 2000. Heterologous protein expression in the methylotrophic yeast Pichia pastoris. Review FEMS Microbiol. Rev. 24: 45-66).

WO2008073914 offenbart die Verwendung eines Peptids, dessen Aminosäuresequenz der N-terminalen Teilsequenz eines Hydrophobins entspricht, als Sekretionssignal, wobei das Peptid in der Lage ist, die Sekretion eines heterologen Fusionsproteins aus einer eukaryotischen Wirtszelle (Chrysosporium) zu vermitteln.

US2004002136 und WO0179558 offenbaren weitere Sekretionssysteme zur Sekretion eines heterologen Fusionsproteins.

Nakari-Setälä *et al.* (1996) offenbart eine Nukleotidsequenz aus *Trichoderma reesei,* die für ein Peptid aus der Signalsequenz des Hydrophobins HFBI und dem maturen Hydrophobin HFBI kodiert. Ferner ist aus Nakari-Setälä et al. (1997) ein Peptid bekannt, das sowohl die Signalsequenz des Hydrophobins HFBII als auch das mature Hydrophobin HFBII umfasst.

Alternativen zu den bereits bekannten Strategien zur Sekretion sind wünschenswert, insbesondere solche, welche auf möglichst kurzen Signalpeptiden beruhen, da diese die Klonierung und Expression der entsprechenden Fusionsproteine erleichtern.

Aufgabe der vorliegenden Erfindung ist es, neue Sekretionssignale anzugeben, mit deren Hilfe die Ausschleusung bzw. der Transport von rekombinant exprimierten Proteinen aus eukaryotischen Zellen, insbesondere Hefen wie *Pichia pastoris,* aber auch Säugerzellen, in das Kulturmedium in vielen verschiedenen Zelltypen mit hoher Effizienz vermittelt wird.

Erfindungsgemäß wird die Aufgabe gelöst durch die Verwendung eines Peptids wie in den Ansprüchen erwähnt.

Hydrophobine stellen eine Familie von oberflächenaktiven Proteinen dar, welche von filamentösen Pilzen gebildet werden. Bemerkenswert ist ihre Eigenschaft, selbstständig an hydrophil/hydrophoben Grenzflächen in robusten Schichten zu assemblieren. Hydrophobine sind in der Lage, hydrophile Oberflächen hydrophob zu machen und umgekehrt. Ferner verringern Hydrophobine die Oberflächenspannung an der Grenzschicht gasförmig/flüssig.

Im Sinn der vorliegenden Erfindung ist eine N-terminale Teilsequenz eines Hydrophobins eine Signalsequenz des Hydrophobins, welche die freie, nicht an einer Peptidbindung beteiligte Aminogruppe umfasst.

Die Erfinder haben überraschend gefunden, dass unter der Verwendung von eukaryotischen Wirtszellen Fusionsproteine, die an ihrem N-Terminus eine N-terminale Teilsequenz aus einem Hydrophobin aus *Trichoderma reesei* enthalten, mit Hilfe eines erfindungsgemäßen Sekretionssignals effizient durch den Sekretionsapparat der Wirtszelle geleitet und schließlich aus der Wirtszelle in das Kulturmedium sezerniert werden.

Insbesondere überraschend war dabei, dass die in den Ansprüchen definierten Sekretionssignale die Sekretion auch aus anderen Wirtsorganismen als *Trichoderma reesei,* aus dem die Sekretionssignale ursprünglich stammen, vermitteln, da solche Sekretionssignale üblicherweise speziesspezifisch wirken. Vorzugsweise vermitteln die erfindungsgemäßen Sekretionssignale daher die Sekretion von Fusionsproteinen aus Wirtszellen, die keine *Trichoderma* reesei-Zellen sind.

Vorzugsweise ist das Hydrophobin aus *Trichoderma reesei* ausgewählt aus den Proteinen HFBI (Genbank Accession No. P52754.1) und HFBII (Genbank Accession No. P79073.1).

Bevorzugt umfasst die N-terminale Teilsequenz wenigstens die zehn N-terminalen Aminosäuren der Aminosäuresequenz von HFBI (SEQ ID No. 1) oder wenigstens die fünfzehn N-terminalen Aminosäuren der Aminosäuresequenz von HFBII (SEQ ID No. 14) oder eine funktionellen Variante davon mit einer Sequenzidentität von mindestens 80 %, bevorzugt mindestens 90 %.

Besonders bevorzugt ist die N-terminale Teilsequenz ausgewählt aus einer der Sequenzen der SEQ ID No. 1 bis 14 oder einer funktionellen Variante davon mit einer Sequenzidentität von mindestens 80 %, bevorzugt mindestens 90 %.

| | |
|---|---|
| SEQ ID No. 1 | MKFFAIAALF |
| SEQ ID No. 2 | MKFFAIAALF A |
| SEQ ID No. 3 | MKFFAIAALF AA |
| SEQ ID No. 4 | MKFFAIAALF AAA |
| SEQ ID No. 5 | MKFFAIAALF AAAA |
| SEQ ID No. 6 | MKFFAIAALF AAAAV |
| SEQ ID No. 7 | MKFFAIAALF AAAAVA |
| SEQ ID No. 8 | MKFFAIAALF AAAAVAQ |
| SEQ ID No. 9 | MKFFAIAALF AAAAVAQP |
| SEQ ID No. 10 | MKFFAIAALF AAAAVAQPL |
| SEQ ID No. 11 | MKFFAIAALF AAAAVAQPLE |
| SEQ ID No. 12 | MKFFAIAALF AAAAVAQPLE D |
| SEQ ID No. 13 | MKFFAIAALF AAAAVAQPLE DR |
| SEQ ID No. 14 | MQFFAVALFA TSALA |

Im Zusammenhang mit den Signalsequenzen werden unter funktionellen Varianten im Sinne dieser Erfindung Aminosäuresequenzen mit einer Sequenzidentität von mindestens 80 % wie in den Ansprüchen definiert, bevorzugt mindestens 90%, verstanden, die als Sekretionssignal geeignet sind, d. h. welche die Ausschleusung von heterolog exprimierten Fusionsproteinen aus eukaryotischen Zellen, bevorzugt Hefen, besonders bevorzugt *Pichia pastoris,* vermitteln. Insbesondere allele Varianten sind von dem Begriff der funktionellen Variante eingeschlossen.

Bevorzugt sind Sequenzen mit den SEQ ID No. 1 bis 10, besonders bevorzugt Sequenzen mit den SEQ ID No. 1 bis 5, ganz besonders bevorzugt Sequenzen mit den SEQ ID No. 1 bis 3, oder funktionelle Varianten davon mit einer Sequenzidentität von mindestens 80 %, bevorzugt mindestens 90 %.

Ein Fusionsprotein im Sinne der vorliegenden Erfindung ist ein Polypeptid wie in den Ansprüchen definiert, das wenigstens zwei Abschnitte aufweist, die in der Natur nicht in Form von aneinander fusionierten Polypeptiden vorkommen. Die Erzeugung eines erfindungsgemäßen Fusionsproteins erfolgt *in vitro,* aber auch *in vivo,* durch bekannte molekularbiologische Methoden. Zu diesem Zweck werden die Protein-kodierenden Abschnitte zweier oder mehrerer Nukleinsäuren im übereinstimmenden Leseraster miteinander verbunden und als Hybrid- oder Fusionsgen in einer geeigneten Wirtszelle oder einem geeigneten zellfreien Expressionssystem exprimiert. Dazu werden vorzugsweise übliche Expressionsvektoren verwendet, die für das erfindungsgemäße Fusionsprotein kodieren. Diese Expressionsvektoren werden in eine geeignete Zelle oder ein geeignetes zellfreies System eingebracht, in der bzw. dem dann das Fusionsprotein produziert wird.

Zweckmäßigerweise umfasst ein Fusionsprotein im Sinne der Erfindung wenigstens das erfindungsgemäße Sekretionssignal und ein zu exprimierendes Protein (Zielprotein) wie in den Ansprüchen definiert.

Unter Zielprotein wird im Zusammenhang mit der Erfindung neben einem Volllängenprotein auch ein Proteinfragment, eine Proteindomäne, ein Hybridprotein oder ein Peptid verstanden.

Da Fusionsproteine per Definition artifiziell erzeugt werden und daher in dieser Form in der Natur nicht vorkommen, werden sie in Zellen üblicherweise heterolog exprimiert, d. h. die Fusionsproteine werden in Wirtszellen erzeugt, die solche Fusionsproteine natürlicherweise nicht produzieren würden. Die natürlich vorkommenden Hydrophobine aus *Trichoderma reesei* sind daher keine Fusionsproteine im Sinne der Erfindung.

Die neuen Sekretionssignale, welche von den Proteinen HFBI (gelegentlich auch als Hydrophobin 1 bezeichnet) bzw. HFBII (Hydrophobin 2) aus *Trichoderma reesei* abgeleitet sind, besitzen die Funktion einer Signalsequenz mit der Fähigkeit, ein Zielprotein, an welches die Signalsequenz N-terminal fusioniert ist; effektiv aus einer Zelle auszuschleusen. Die erfindungsgemäßen, von HFBI bzw. HFBII abgeleiteten Sekretionssignale leiten die Sekretion von Zielproteinen in eukaryotischen Zellen, bevorzug in Hefen, ein, wenn diese Signalsequenzen N-terminal an ein Zielprotein, wie z. B. Enhanced Green Fluorescent Protein (EGFP) fusioniert werden. Die Stärke der Sekretion ist vergleichbar mit derjenigen des kommerziell erhältlichen α-Faktors-Signalpeptides.

Durch die Verwendung des Sekretionssignals wie in den Ansprüchen definiert werden Proteine effizient und in hohem Maß in den Kulturüberstand abgegeben. Da Hefen *per se* nur geringe Mengen Protein ausschleusen, ist eine Aufreinigung eines Proteins aus dem Kulturüberstand vorteilhaft wesentlich einfacher und schneller als aus dem Lysat der Zellen. Außerdem entfalten die Sekretionssignale ihre sekretorische Wirkung vorteilhaft in verschiedenen eukaryotischen Wirtszellen, insbesondere in Hefen.

Das Sekretionssignal wie in den Ansprüchen definiert wird üblicherweise während der Sekretion aus der Wirtszelle abgespalten, so dass vorteilhafterweise nur das Zielprotein aus der Wirtszelle in das umgebende Medium abgegeben wird. Die Sekretion von Proteinen ist jedoch nicht obligat an die Abspaltung des Sekretionssignals gekoppelt. So ist es nicht zwingend notwendig, dass in jeder Art von Wirtszelle, in der das Fusionsprotein exprimiert wird, das Sekretionssignal bei der Sekretion des Fusionsproteins abgespalten wird.

Wird eine Wirtszelle zur Expression des Fusionsproteins eingesetzt, in welcher keine Abspaltung des Sekretionssignals während der Sekretion erfolgt, so kann das Zielprotein durch die Verwendung einer artifiziellen Proteaseschnittstelle erhalten werden. Diese wird zwischen das Sekretionssignal und das Zielprotein kloniert. Dadurch kann das Sekretionssignal nach der Aufreinigung durch eine geeignete Protease von dem Zielprotein abgespalten werden.

Das Sekretionssignal wie in den Ansprüchen definiert eignet sich besonders für die rekombinante Herstellung von glykosylierten Zielproteinen. Eukaryotische Proteine sind häufig glykosyliert, d. h. sie werden nach der Translation durch spezifische Enzyme mit Zuckern verknüpft. Die Glykosylierung spielt sowohl für die Konformation als auch für die Funktion vieler eukaryotischer Proteine des sekretorischen Pathways eine bedeutende Rolle. Da Prokaryonten über kein vergleichbares Glykosylierungssystem verfügen, werden glykosylierte Proteine regelmäßig in eukaryotischen Wirtszellen exprimiert. Dies allein genügt jedoch nicht, um eine Glykosylierung zu gewährleisten. Da die Glykosylierung ausschließlich im sekretorischen Pathway stattfindet, muss sichergestellt werden, dass das rekombinant exprimierte Zielprotein auch in den sekretorischen Pathway gelangt, damit es glykosyliert werden kann. Dies ist durch die Fusionierung des Zielproteins mit dem Sekretionssignal wie in den Ansprüchen definiert vorteilhaft möglich.

Die Herstellung von Fusionsproteinen aus Zielprotein und Sekretionssignal erfolgt mit dem Fachmann geläufigen molekularen Methoden. Dazu wird ein für das Zielprotein kodierendes Nukleinsäuremolekül unter der Beachtung des Leserahmens derart an das 3'-Ende eines für ein Sekretionssignal wie in den Ansprüchen definiert kodierendes Nukleinsäuremolekül kloniert, dass ein Nukleinsäuremolekül entsteht, welches für ein Fusionsprotein kodiert, das an seinem N-Terminus das Sekretionssignal wie in den Ansprüchen definiert und C-terminal davon das Zielprotein enthält.

Bestandteil der Erfindung sind daher auch die für ein Sekretionssignal wie in den Ansprüchen definiert gemäß einer der Sequenzen mit den SEQ ID No. 1 bis 14 oder einer funktionellen Variante davon kodierenden Nukleinsäuremoleküle.

Bevorzugt sind dies die Nukleinsäuremoleküle gemäß den SEQ ID No. 15 bis 28.

| | |
|---|---|
| SEQ ID No. 15 | ATG AAG TTC TTC GCC ATC GCC GCT CTC TTT |
| SEQ ID No. 16 | ATG AAG TTC TTC GCC ATC GCC GCT CTC TTT GCC |
| SEQ ID No. 17 | ATG AAG TTC TTC GCC ATC GCC GCT CTC TTT GCC GCC |
| SEQ ID No. 18 | ATG AAG TTC TTC GCC ATC GCC GCT CTC TTT GCC GCC GCT |
| SEQ ID No. 19 | ATG AAG TTC TTC GCC ATC GCC GCT CTC TTT GCC GCC GCT GCC |
| SEQ ID No. 20 | |
| SEQ ID No. 21 | |
| SEQ ID No. 22 | |
| SEQ ID No. 23 | |
| SEQ ID No. 24 | |
| SEQ ID No. 25 | |
| SEQ ID No. 26 | |
| SEQ ID No. 27 | |
| SEQ ID No. 28 | |

Besonders bevorzugt sind die Sequenzen mit den SEQ ID No. 15 bis 24, ganz besonders bevorzugt die Sequenzen mit den SEQ ID No. 15 bis 19.

Aufgrund der Degeneration des genetischen Codes existieren zahlreiche weitere Nukleinsäuremoleküle, welche für die in den Ansprüchen definierten Sekretionssignale kodieren und die ebenfalls von der Erfindung umfasst sind und für den Fachmann leicht ableitbar sind.

Bestandteil der Erfindung sind desweiteren Fusionsproteine, welche ein erfindungsgemäßes Sekretionssignal und ein Zielprotein umfassen, sowie die für solche Fusionsproteine kodierenden Nukleinsäuremoleküle. Das Fusionsprotein enthält gegebenenfalls weitere Abschnitte, wie z. B. eine Proteaseschnittstelle oder einen Proteintag, die sich entweder zwischen dem Sekretionssignal und dem Zielprotein oder C-terminal vom Zielprotein befinden können.

Das 5'-Ende der für das Zielprotein kodierenden Nukleinsäure muss daher nicht direkt und unmittelbar an das 3'-Ende des für das Sekretionssignal wie in den Ansprüchen definiert kodierenden Nukleinsäuremoleküls kloniert sein. Gegebenenfalls befinden sich dazwischen noch andere Nukleinsäureabschnitte, die entweder die Funktion eines Linkers, d. h. eines Verbindungsstücks, übernehmen oder die für funktionelle Aminosäuresequenzen kodieren, welche im zu exprimierenden Fusionsprotein bestimmte Aufgaben erfüllen, beispielsweise eine Proteaseschnittstelle oder eine Antikörperbindungsstelle (z. B. Myc-Tag, HA-Tag).

Weiterhin können die erfindungsgemäßen Fusionsproteine posttranslational modifiziert, z. B. glykosyliert, phosphoryliert oder acetyliert, sein.

Vorteilhaft sind die neu entwickelten Sekretionssignale sehr kurz und durch einen geeigneten Primer artifiziell vor jedes beliebige Nukleinsäuremolekül und in jeden beliebigen Vektor klonierbar. Sie sind somit molekularbiologisch extrem einfach handhabbar.

Bestandteil der Erfindung ist daher auch die Verwendung eines Oligonukleotids, welches ein Nukleinsäuremolekül umfasst, das zu einem für ein Sekretionssignal gemäß den SEQ ID No. 1 bis 13 kodierenden Nukleinsäuremolekül komplementär ist, als Primer in einer Polymerasekettenreaktion (PCR, *Polymerase chain reaction).*

Mittels der Polymerasekettenreaktion können Nukleinsäuremoleküle amplifiziert, d. h. vermehrt, werden. Primer enthalten eine Nukleotidsequenz, die zu einem flankierenden Abschnitt des zu amplifizierenden Nukleinsäuremoleküls komplementär ist, und dienen in der Polymerasekettenreaktion nach Hybridisation mit der zu amplifizierenden Nukleotidsequenz als Startpunkt für die Nukleinsäure-replizierenden Enzyme, wie z. B. die DNA-Polymerase.

Um ein für ein erfindungsgemäßes Fusionsprotein kodierendes Nukleinsäuremolekül zu erzeugen, umfasst ein solches Oligonukleotid bevorzugt zusätzlich eine mindestens 12 Nukleotide lange Sequenz, welche zu dem 5'-Ende des zu amplifizierenden, für das Zielprotein kodierenden Nukleinsäuremoleküls komplementär ist und die Hybridisierung des Primers mit der zu amplifizierenden Sequenz erlaubt.

Gegebenenfalls enthält das Oligonukleotid Schnittstellen für Restriktionsenzyme, um nach abgeschlossener Polymerasekettenreaktion die Klonierung des amplifizierten Nukleinsäuremoleküls in einen Klonierungsvektor oder Expressionsvektor bzw. eine Expressionskassette zu ermöglichen.

Das Oligonukleotid ist bevorzugt entweder eine Desoxyribonukleinsäure, eine Ribonukleinsäure oder eine andere Nukleinsäure, beispielsweise mit einem artifiziellen Rückgrat, wie z. B. Peptidnukleinsäure (PNA) oder Morpholino.

Das Fusionsprotein aus Sekretionssignal und Zielprotein wird nach der Expression in der Wirtszelle in das die Wirtszelle umgebende Medium transportiert. Dazu muss die für dieses Fusionsprotein kodierende Nukleinsäure in einer Wirtszelle exprimiert werden. Gegenstand der Erfindung ist daher auch eine Expressionskassette, ein Expressionsvektor oder ein artifizielles Chromosom, die eine für ein erfindungsgemäßes Fusionsprotein kodierende Nukleinsäuremolekül umfassen.

Artifizielle Chromosomen sind Vektoren, die natürlichen Chromosomen nachgebildet sind und insbesondere zur genetischen Modifikation von Hefen verwendet werden. Sie umfassen Sequenzelemente, welche für die Stabilität und Vermehrung des Chromosoms notwendig sind: Replikationsursprungsorte, ein Centromer, das die Verteilung auf die Tochterzellen gewährleistet, und Telomersequenzen, welche die Enden des artifiziellen Chromosoms bilden.

Eine erfindungsgemäße Expressionskassette, ein erfindungsgemäßer Expressionsvektor oder ein erfindungsgemäßes artifizielles Chromosom enthält zweckmäßigerweise einen Promotor, d. h. eine Nukleotidsequenz, welche die regulierte Expression der für das erfindungsgemäße Expressionsprotein kodierenden Nukleinsäure ermöglicht. Die erfindungsgemäßen Expressionskassetten, Expressionsvektoren oder artifiziellen Chromosomen enthalten bevorzugt weitere funktionale Sequenzbereiche, wie z. B. eine Replikationsstartpunkt, Operatoren, Terminationssignale, oder für Selektionsmarker, Repressoren oder Aktivatoren kodierende Nukleotidsequenzen.

Gegenstand der Erfindung ist auch eine eukaryotische Wirtszelle, die eine erfindungsgemäße Expressionskassette, einen erfindungsgemäßen Expressionsvektor oder ein erfindungsgemäßes artifizielles Chromosom umfasst. Der Begriff Wirtszelle umfasst transient (d. h. vorübergehend) transfizierte Zellen (z. B. durch mRNA), plasmidtransformierte Wirtszellen oder artifizielle Chromosomen enthaltende Wirtszellen sowie auch Wirtszellen, in denen die erfindungsgemäße Expressionskassette oder der Expressionsvektor stabil in das Genom integriert ist. Der Begriff Wirtszelle umfasst keine humanen embryonalen Stammzellen.

Bevorzugte Wirtszellen sind Hefen, insbesondere *Pichia pastoris.* Aber auch die Verwendung anderer dem Fachmann geläufiger Hefen, wie z. B. *Saccharomyces cerevisiae, Schizosaccharomyces pombe* und *Hansenula polymorpha* ist möglich. Außerdem sind Zellinien höherer Eukaryonten, wie z. B. HeLa-Zellen (Zellen des Zervix-Karzinoms) als Wirtszellen geeignet.

Gegenstand der vorliegenden Erfindung ist weiterhin die Verwendung einer erfindungsgemäßen Expressionskassette, eines erfindungsgemäßen Expressionsvektors oder eines erfindungsgemäßen artifiziellen Chromosoms zur Transformation oder Transfektion von eukaryotischen Wirtszellen sowie die Verwendung der so erzeugten eukaryotischen Wirtszellen zur rekombinanten Expression eines erfindungsgemäßen Fusionsproteins.

Anhand nachfolgender Figuren und Ausführungsbeispiele wird die Erfindung näher erläutert, ohne diese einzuschränken.
**Figur 1** zeigt einen Western Blot. EGFP, welches C-terminal mit einem c-myc-Tag markiert war, wurde mittels eines Anti-c-myc-Antikörpers detektiert. Fig. 1 a) zeigt Proteine nach Zellaufschluss, Fig. 1 b) Proteine aus dem Kultur-Überstand (1.8 mL der TCA-Fällung).
**Fig. 2** zeigt die Mittelwerte der Fluoreszenz, die intrazellulär in *Pichia pastoris* mittels Flowzytometer gemessen wurde. In Zellen, in welchen EGFP mit dem erfindungsgemäßen Sekretionssignal fusioniert war, ist das intrazelluläre Fluoreszenzsignal im Vergleich zu Zellen, die EGFP ohne Sekretionssignal exprimierten, deutlich reduziert.
**Fig. 3** zeigt die Mittelwerte der Fluoreszenz, die intrazellulär in *S. cerevisiae* mittels Flowzytometer gemessen wurde.
**Fig. 4** zeigt eine mikroskopische Aufnahme der *S. cerevisiae* Kontrollstämme (alpha-EGFP = Kontrollstamm für Sekretion, EGFP = Kontrollstamm für intrazelluläre Expression).
**Fig. 5** zeigt eine mikroskopische Aufnahme der *S. cerevisiae* Stämme, welche EGFP fusioniert mit der kurzen (K-EGFP) und der langen (L-EGFP) Signalsequenz des HFBI exprimieren.
**Fig. 6** zeigt eine mikroskopische Aufnahme eines *S. cerevisiae* Stamms, welcher EGFP fusioniert mit der Signalsequenz des HFBII exprimiert.
**Fig. 7** zeigt die Mittelwerte der Fluoreszenz, die intrazellulär in *S. pombe* mittels Flowzytometer gemessen wurde.
**Fig. 8** zeigt eine mikroskopische Aufnahme eines *S*. *pombe* Stamms mit intrazellulärer EGFP-Expression.
**Fig. 9** zeigt mikroskopische Aufnahmen von *S*. *pombe* Stämmen, die EGFP exprimieren, welches mit der kurzen (K-EGFP) Signalsequenz des HFBI fusioniert ist.
**Fig.10** zeigt mikroskopische Aufnahmen von *S*. *pombe* Stämmen, die EGFP exprimieren, welches mit der langen (L-EGFP) Signalsequenz des HFBI fusioniert ist.
**Fig. 11** zeigt mikroskopische Aufnahmen eines *S*. *pombe* Stammes, der EGFP exprimiert, das mit der Signalsequenz des HFBII (h2-EGFP) fusioniert ist.
**Fig. 12** zeigt eine mikroskopische Untersuchung von HeLa-Zellen, welche EGFP exprimieren, das mit der langen Signalsequenz des HFBI (L-EGFP) bzw. mit keiner Signalsequenz (EGFP) fusioniert ist.

### Ausführungsbeispiele:

Auf experimenteller Ebene wurden DNA-Konstrukte mittels molekularbiologischer Methoden hergestellt, welche für Fusionsproteine kodieren, die N-terminal eines der angegebenen Sekretionssignale enthalten:
L- Sekretionssignal (lang) SEQ ID No. 27
K-Sekretionssignal (kurz) SEQ. ID No. 21
   5`-ATGAAGTTCTTCGCCATCGCCGCTCTCTTTGCCGCCGCTGCCGTTGCC-3`
h2-EGFP (Sekretionssignal HFBII) SEQ. ID No. 28
   5'-ATGCAGTTCTTCGCCGTCGCCCTCTTCGCCACCAGCGCCCTGGCT-3'

Als beispielhaftes Zielprotein diente EGFP. Die entsprechenden Konstrukte wurden mit den jeweiligen Negativkontrollen (EGFP ohne Sekretionssignal) und ggf. auch Positivkontrollen (alpha-EGFP = EGFP, das mit dem kommerziell erhältlichen alpha-Faktor-Signalpeptid fusioniert war) in die jeweiligen Hefen (*Pichia pastoris, Saccharomyces cerevisiae* und *Schizosaccharomyces pombe*) sowie in humane HeLa Zellen transformiert.

### Beispiel 1: Sekretion von Fusionsprotein aus der Hefe Pichia pastoris

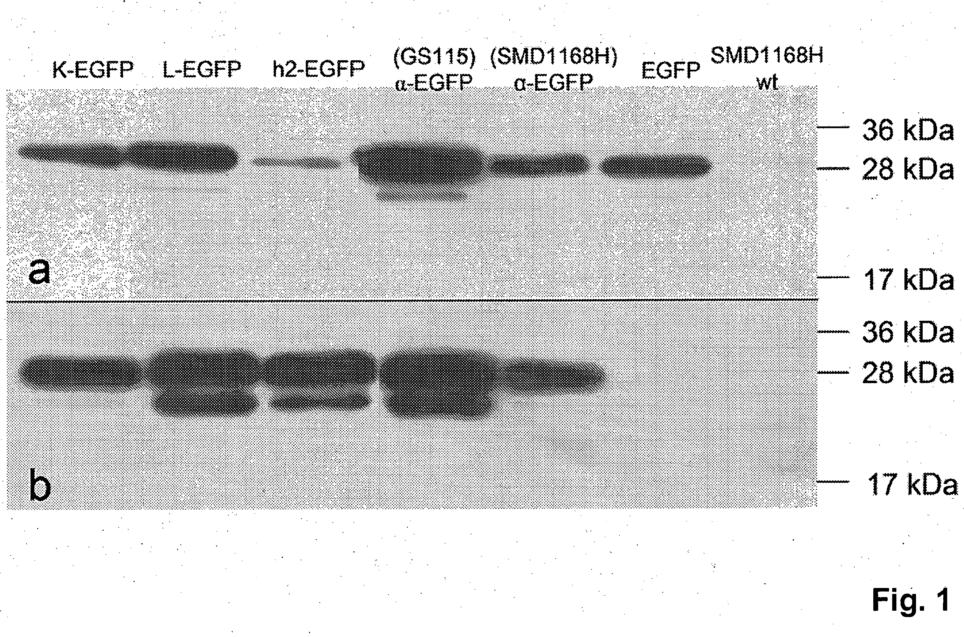

In Fig. 1 ist ein Western-Blot mit anschließender immunologischer Detektion (c-Myc-Tag am EGFP) dargestellt. Dieser Blot verdeutlicht das Potential der neu entdeckten Sekretionssequenzen. Der Überstand der Kultur wurde nach der Kultivierung der Stämme durch Zentrifugation von den Zellen getrennt. Im Teil a der Fig. 1 ist der in den Zellen befindliche EGFP-Anteil dargestellt. Es wurden jeweils 20 µg Proteine des Lysats der Zellen aufgetragen. Teil b dokumentiert die in den Kulturüberstand sezernierten Mengen an EGFP. Es wurden die aus dem Kulturüberstand gefällten Proteine aufgetragen.

Als Kontrolle für die Expression von sezerniertem EGFP wurde das Reporterprotein EGFP mit dem kommerziellen α-Faktor fusioniert und in den Hefestämmen *Pichia pastoris* SMD1168H und *Pichia pastoris* GS115 unter der Kontrolle des GAP-Promotors exprimiert und analysiert (alpha-EGFP; Spur 4 und 5).

Wie in Fig. 1 zu erkennen ist, wird EGFP in den Zellen hergestellt (Fig.1 a) und effektiv ausgeschleust (Fig. 1 b). Der Kontrollstamm (*Pichia pastoris* GS115) für die Expression von nicht-sezerniertem EGFP (Spur 6), in dem EGFP ohne ein erfindungsgemäßes Sekretionssignal unter der Kontrolle des GAP-Promotors exprimiert wird, zeigt im Gegensatz dazu kein Signal in Teil b, sondern nur in Fig. 1a, also im Lysat der Zellen.

Die Spur 7 zeigt als Kontrolle die entsprechenden Fraktionen von Wildtyp-Zellen des Stammes *Pichia pastoris* SMD 1168H, welche weder im Lysat der Zellen noch im Überstand ein Signal zeigen.

Spur 1 zeigt den Stamm, der die kurze Signalsequenz (K) am N-Terminus des EGFP trägt. Hier ist im Lysat der Zellen (Fig.1 a) und auch im Überstand (Fig. 1 b) ein deutliches Signal zu erkennen. Das Sekretionssignal führt offensichtlich zu einer sehr effektiven Sekretion von EGFP. Auch im Fall der Zellen, in denen die lange Signalsequenz (L) mit EGFP fusioniert ist (Spur 2), oder im Fall des h2-EGFP Fusionskonstruktes (Spur 3), ist zu erkennen, dass sich ein hoher Anteil an gebildetem EGFP außerhalb der entsprechenden Zellen befindet.

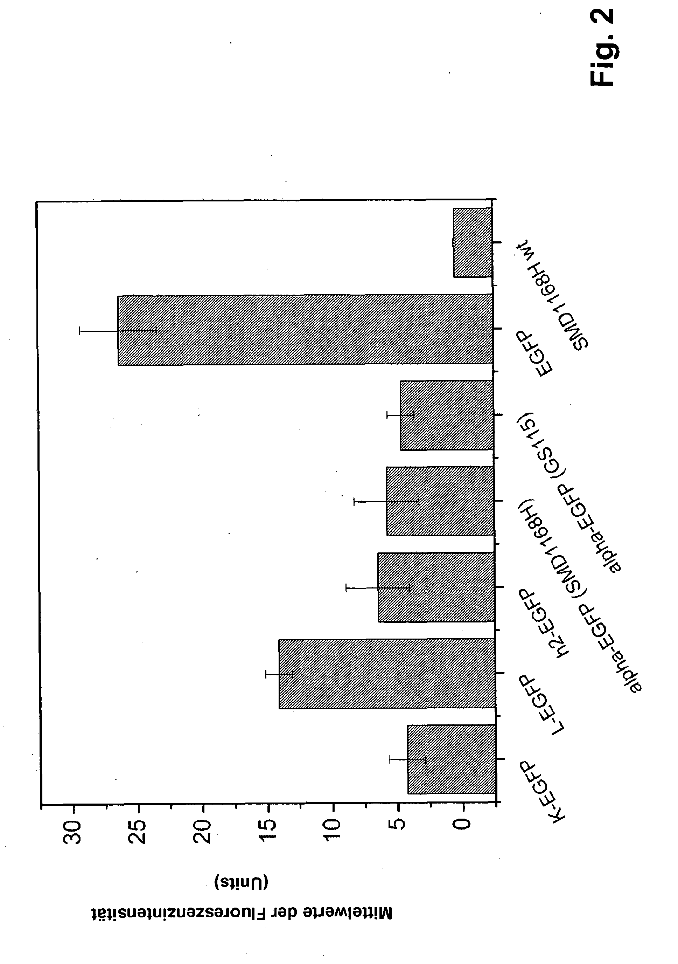

Die Ergebnisse der Analysen mittels Flowzytometer (Fig. 2) zeigen im Fall des intrazellulär exprimierenden Kontrollstamms (EGFP ohne Sekretionssignal) ein starkes Fluoreszenzsignal, welches vom EGFP in den Zellen ausgelöst wird.

Im Fall der Kontrollstämme für Sekretion (alpha-EGFP) ist hingegen eine deutlich geringere Fluoreszenzintensität messbar, welches auf ein effizientes Ausschleusen des Zielproteins aus den Zellen hindeutet. Die von HFBI abgeleiteten Signalsequenzen (L-EGFP und K-EGFP) sowie die vom HFBII abgeleitete Signalsequenz (h2-EGFP) zeigen ebenfalls in der Flowzytometer-Messung sehr geringe Fluoreszenzintensitäten, welche auf eine Sekretion des Zielproteins EGFP in den Kulturüberstand schließen lassen.

Beide Sekretionssignale L und K, abgeleitet vom Hydrophobin HFBI aus *Trichoderma reesei,* sowie das h2-Sekretionssignal, abgeleitet vom Hydrophobin HFBII aus *Trichoderma reesei,* sind in der Hefe *Pichia pastoris* bezüglich ihrer Effizienz mit dem kommerziell erhältlichen αFaktor vergleichbar.

### Ausführungsbeispiel 2: Sekretion von Fusionsprotein aus der Hefe Saccharomyces cerevisiae

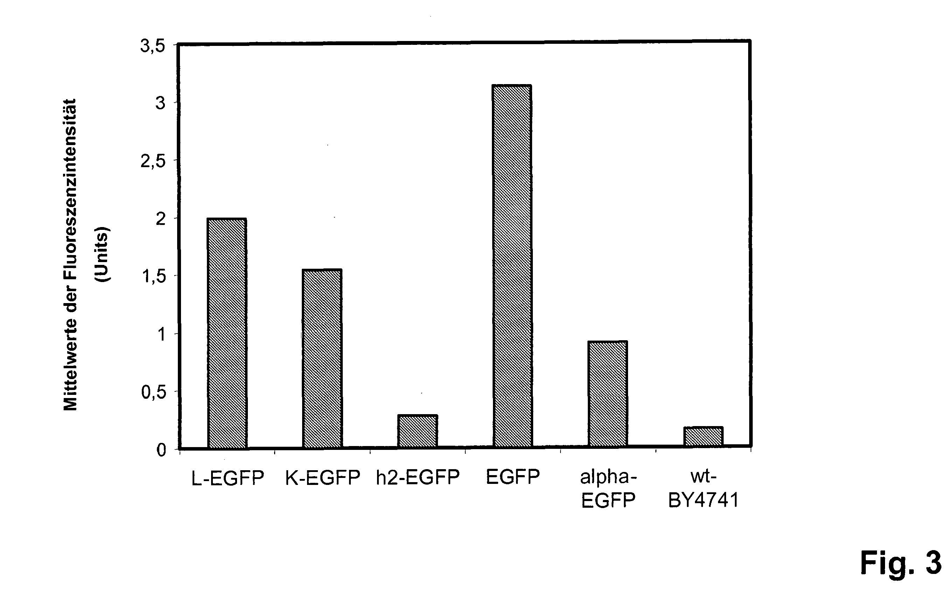

Die Ergebnisse der Flowzytometer-Analyse (Fig. 3) zeigen eine effiziente Ausschleusung des Modellproteins EGFP aus den Zellen. Der Kontrollstamm für die intrazelluläre Expression des EGFP (Fig. 3, EGFP, Säule 4) zeigt ein Fluoreszenzsignal von großer Intensität, wohingegen der Kontrollstamm für Sekretion (Fig. 3, alpha-EGFP, Säule 5) ein geringes Signal zeigt, was auf die Sekretion des EGFP aus der Zelle in das Medium schließen lässt. Als Negativkontrolle wurde der Wildtypstamm wt-BY4741 verwendet, der kein EGFP exprimiert (Fig. 3, EGFP, Säule 6).

Das EGFP verbleibt im Fall des intrazellulär exprimierenden Stamms in der Zelle und akkumuliert dort. Wird EGFP aus der Zelle sezerniert, dann weist diese - wie an dem Kontrollstamm alpha-EGFP zu sehen - nur eine sehr geringe Fluoreszenz auf.

Die Stämme mit den vom HFBI und HFBII abgeleiteten Sekretionssignalen (K-EGFP, L-EGFP und h2-EGFP) liegen in ihrer Fluoreszenzintensität eher im Bereich des Kontrollstamms für Sekretion (alpha-EGFP). Die mikroskopischen Aufnahmen verdeutlichen dies:

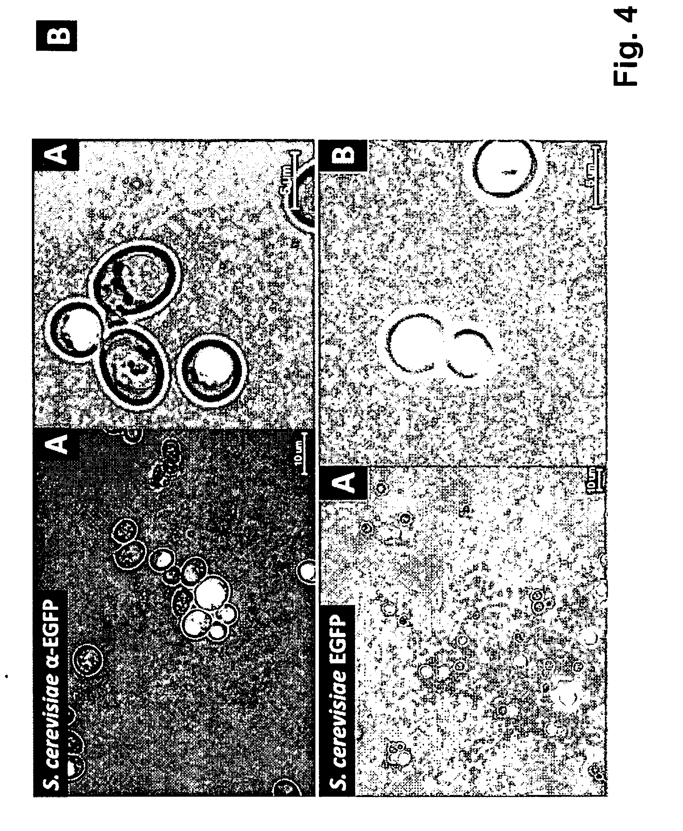

Fig. 4 zeigt die Kontrollstämme. Im Fall des alpha-EGFP Stamms, der das EGFP mit Hilfe des alpha-Faktor-Sekretionssignals sezerniert, ist deutlich zu sehen, dass das EGFP in Vakuolen-ähnlichen Strukturen zu finden ist. Dies legt nahe, dass es in den sekretorischen Pathway der Hefezelle gelangt und ausgeschleust wird. Der Kontrollstamm für intrazelluläre EGFP-Expression (EGFP) zeigt eine diffuse Verteilung des EGFPs im gesamten Cytoplasma.

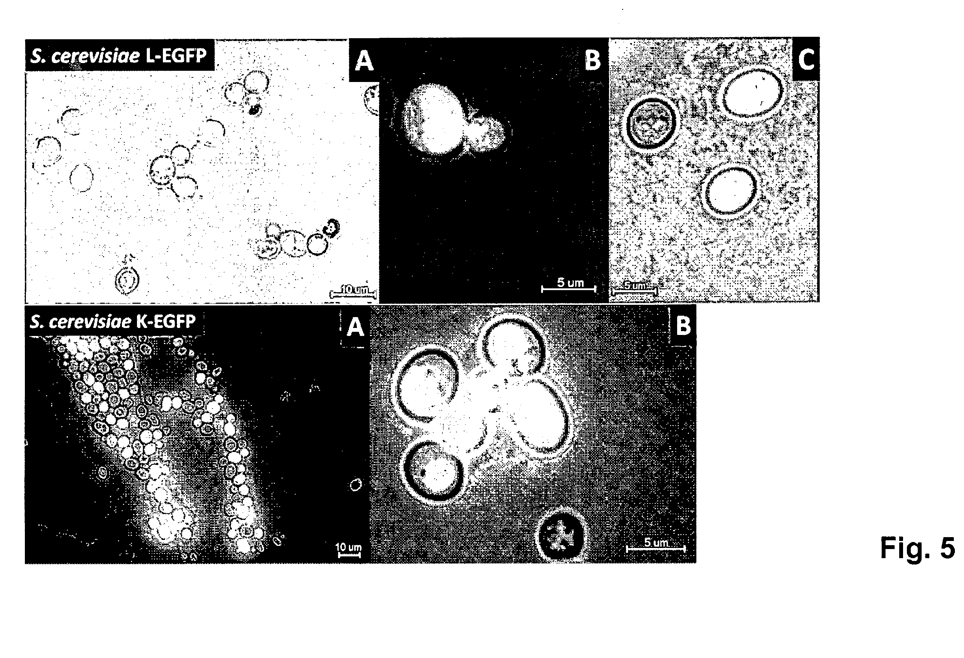

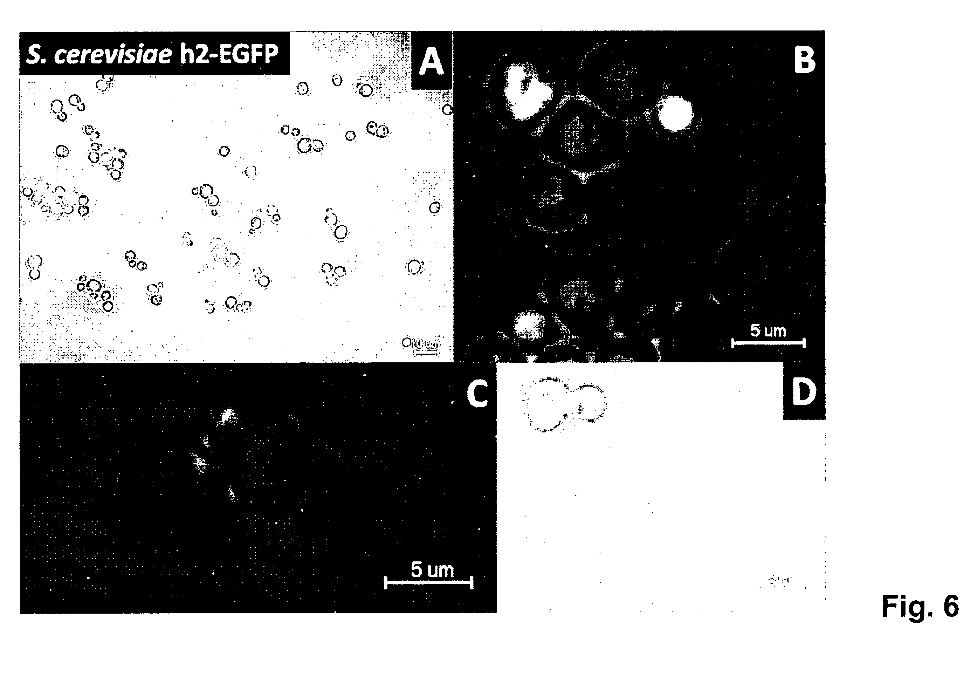

Die Stämme mit den von Hydrophobin abgeleiteten Sekretionssignalen (K-EGFP, L-EGFP und h2-EGFP) sind in Fig. 5 und 6 dargestellt. Das EGFP befindet sich wie im Falle des alpha-EGFP in allen drei Stämmen in Vakuolen-ähnlichen Strukturen der Zellen. Dies deutet auf eine gleiche Prozessierung bzw. Sekretion des EGFP hin.

Die Versuchsergebnisse aus Flowzytometrie und Mikroskopie lassen darauf schließen, dass die neuartigen Sekretionssignale auch im Fall von *S. cerevisiae* zu effizienten Sekretion rekombinanter Proteine führen.

### Ausführungsbeispiel 3: Sekretion aus der Hefe Schizosaccharomyces pombe

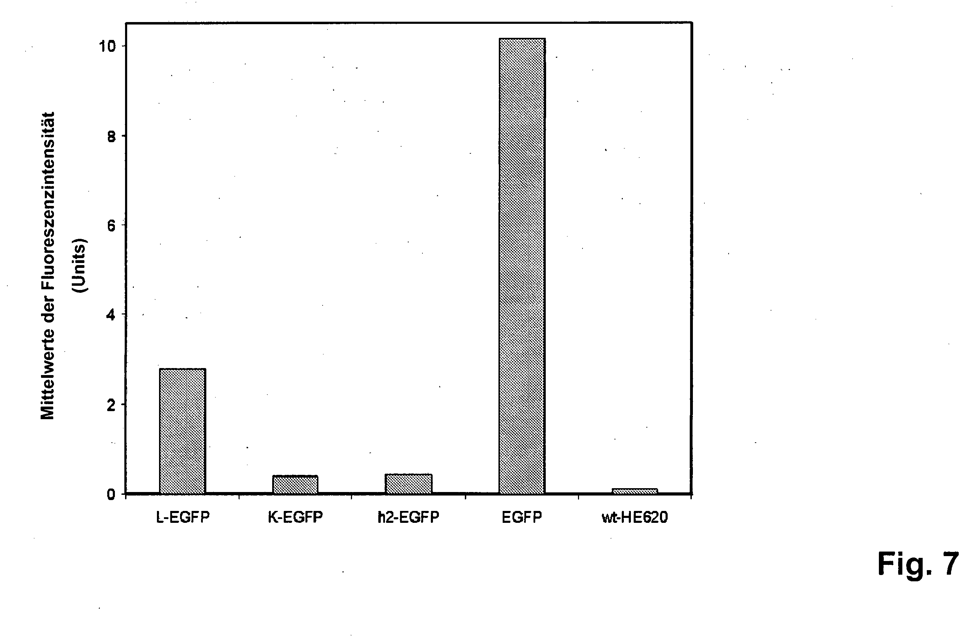

In *S. pombe* wurden analoge Ergebnisse zu den zuvor beschrieben Experimenten mit *S. cerevisiae* erhalten. Sowohl die Ergebnisse der Flowzytometrie-Messungen als auch die der mikroskopischen Untersuchungen zeigen, dass das EGFP in *S. pombe* Stämmen, in denen EGFP fusioniert mit den neuartigen Sekretionssignalen (K-EGFP, L-EGFP und h2-EGFP) exprimiert wird, in den sekretorischen Pathway der Hefe gelangt und effizient aus den Zellen ausgeschleust wird. Es kommt zu einer Verminderung der Fluoreszenzintensität im Vergleich zum intrazellulär exprimierenden Kontrollstamm (EGFP) (Fig. 7). Als Negativkontrolle wurde der Wildtyp-Stamm wt-HE620 verwendet, der kein EGFP exprimiert.

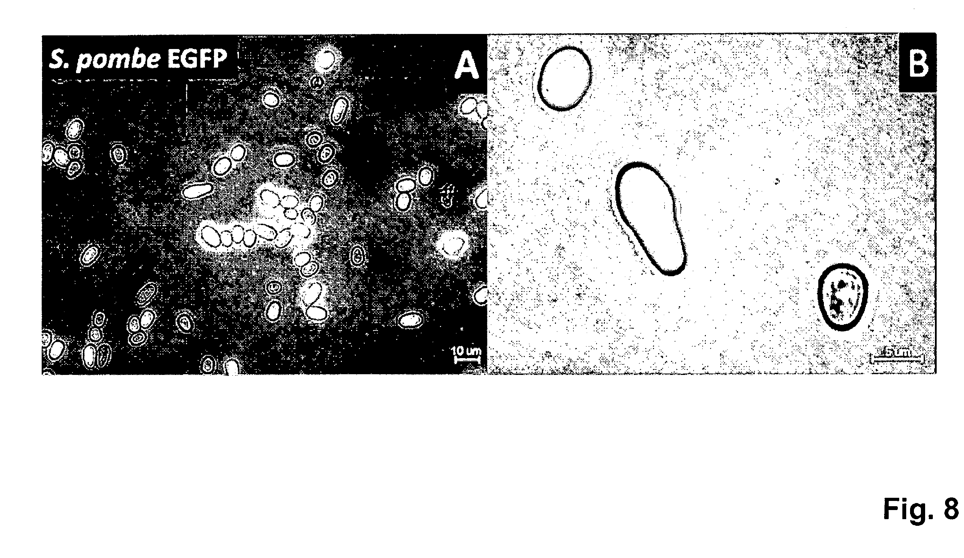

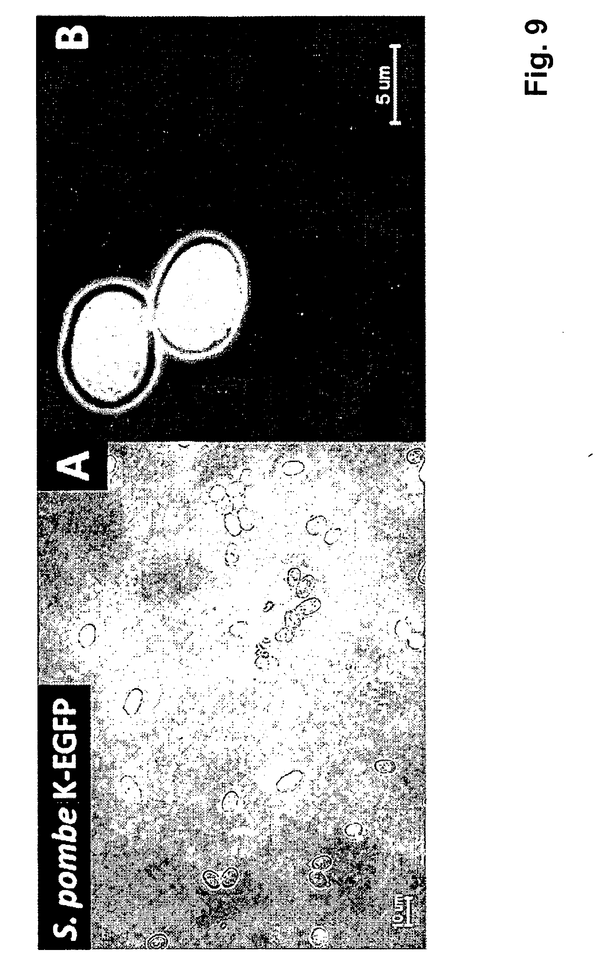

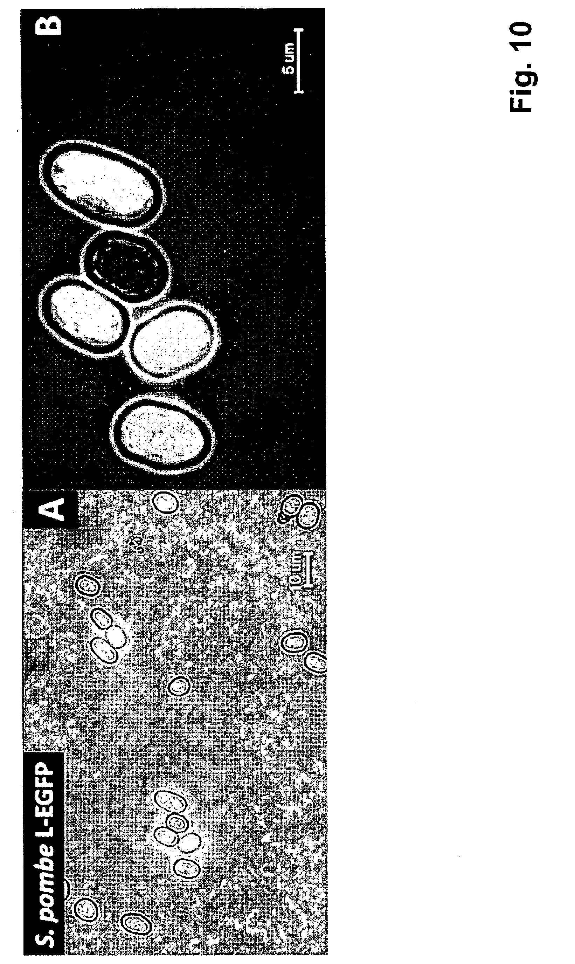

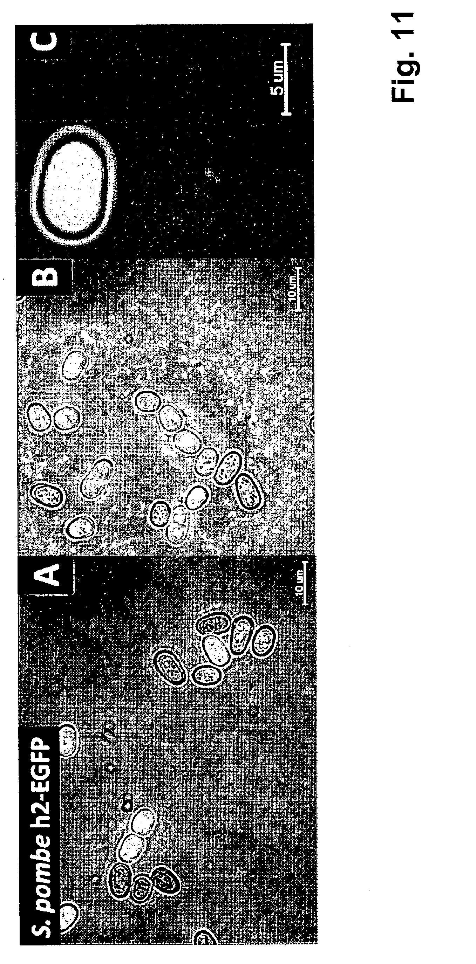

Mikroskopische Aufnahmen zeigen eindeutig dass sich das EGFP in den mit den neuartigen Konstrukten transformierten Zellen (K-EGFP, L-EGFP und h2-EGFP) (Fig. 9 bis 11) in Kompartimenten-ähnlichen Strukturen befindet, während es in der Kontrolle (EGFP) frei im Cytoplasma verteilt ist (Fig. 8). Das lässt den Rückschluss zu, dass EGFP in diesen Zellen in den sekretorischen Pathway gelangt.

### Ausführungsbeispiel 4: Sekretion aus humanen HeLa Zellen

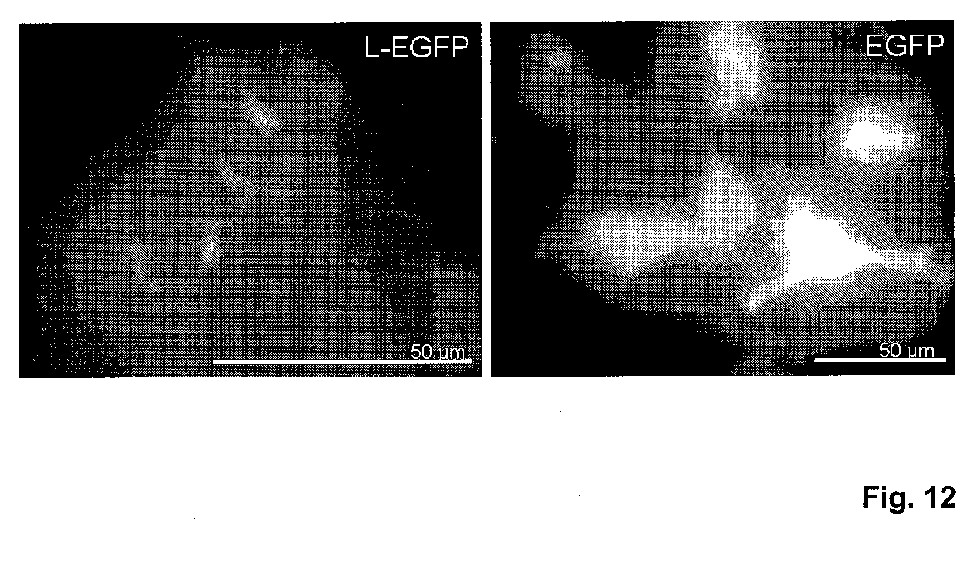

Auch im Fall des humanen Expressionssystems in HeLa Zellen konnte mittels mikroskopischer Untersuchungen (Fig. 12) gezeigt werden, dass die Signalsequenz des HFBI (L-EGFP) zu einer Vermittlung des Zielproteins in den sekretorischen Pathway (EGFP befindet sich in Vesikeln) führt, was auf eine effiziente Ausschleusung des Proteins schließen lässt. Im Vergleich dazu zeigen intrazellulär exprimierende HeLa Zellen eine Lokalisation des EGFPs im gesamten Innenraum der Zellen.

## Patentansprüche

1. Verwendung eines Peptids, dessen Aminosäuresequenz der N-terminalen Signalsequenz eines Hydrophobins aus *Trichoderma reesei* oder einer funktionellen Variante davon entspricht, wobei die funktionelle Variante des Hydrophobins aus *Trichoderma reesei* eine Aminosäuresequenzidentität zur Signalsequenz des Hydrophobins aus *Trichoderma reesei* von mindestens 80 % aufweist, als Sekretionssignal, wobei das Peptid in der Lage ist, die Sekretion eines heterologen Fusionsproteins aus einer eukaryotischen Wirtszelle zu vermitteln.

2. Verwendung nach Anspruch 1, wobei das Hydrophobin ausgewählt ist aus HFBI und HFBII aus *Trichoderma reesei.*

3. Verwendung nach Anspruch 1 oder 2, wobei die Signalsequenz wenigstens die zehn N-terminalen Aminosäuren der Aminosäuresequenz von HFBI oder wenigstens die fünfzehn N-terminalen Aminosäuren der Aminosäuresequenz von HFBII oder eine funktionellen Variante einer dieser Sequenzen mit einer Sequenzidentität von mindestens 80 % umfasst.

4. Verwendung nach einem der Ansprüche 1 bis 3, wobei die Signalsequenz ausgewählt ist aus einer der Sequenzen der SEQ ID No. 1 bis 14 oder einer funktionellen Variante davon mit einer Sequenzidentität von mindestens 80 %.

5. Ein heterologes Fusionsprotein enthaltend ein Peptid wie in einem der Ansprüche 1 bis 4 definiert.

6. Nukleinsäuremolekül kodierend für ein Fusionsprotein gemäß Anspruch 5.

7. Eine Expressionskassette, ein Expressionsvektor oder ein artifizielles Chromosom, umfassend eine Nukleinsäuremolekül gemäß Anspruch 6.

8. Verwendung einer Expressionskassette, eines Expressionsvektors und/oder eines artifiziellen Chromosoms gemäß Anspruch 7 zur Transformation oder Transfektion einer eukaryotischen Wirtszelle.

9. Eine eukaryotische Wirtszelle, umfassend eine Expressionskassette, einen Expressionsvektor und/oder ein artifizielles Chromosom gemäß Anspruch 7.

10. Verwendung einer eukaryotischen Wirtszelle gemäß Anspruch 9 zur Expression eines heterologen Fusionsproteins nach Anspruch 6.

## Claims

1. Use of a peptide, whose amino acid sequence corresponds to the N-terminal sequence of a hydrophobin from *Trichoderma reesei* or a to functional variant thereof, whereby the functional variant of the hydrophobin from *Trichoderma reesei* has an amino acid sequence identity with the signal sequence of the hydrophobin from *Trichoderma reesei* of at least 80%, as a secretion signal, and whereby the peptide is capable of mediating the secretion of a heterologous fusion protein from an eukaryotic host cell.

2. Use according to claim 1, whereby the hydrophobin is selected from HFBI and HFBII from *Trichoderma reesei.*

3. Use according to claim 1 or 2, whereby the signal sequence comprises at least the ten N-terminal amino acids of the amino acid sequence of HFBI or at least the fifteen N-terminal amino acids of the amino acid sequence of HFBII, or of a functional variant of one of these sequences having a sequence identity of at least 80%.

4. Use according to any of the claims 1 to 3, whereby the signal sequence is selected from one of the sequences of the SEQ ID, Nos. 1 to 14, or from a functional variant thereof, having a sequence identity of at least 80 %.

5. A heterologous fusion protein containing a peptide as defined in one of the claims 1 to 4.

6. A nucleic acid molecule coding for a fusion protein according to claim 5.

7. An expression cassette, an expression vector or an artificial chromosome, comprising a nucleic acid molecule according to claim 6.

8. Use of an expression cassette, an expression vector and/or an artificial chromosome, according to claim 7, for transformation or transfection of an eukaryotic host cell.

9. An eukaryotic host cell, comprising an expression cassette, an expression vector and/or an artificial chromosome according to claim 7.

10. Use of an eukaryotic host cell according to claim 9 for the expression of a heterologous fusion protein according to claim 6.

## Revendications

1. Utilisation d'un peptide dont la séquence d'acides aminés correspond à la séquence signal N-terminale d'une hydrophobine provenant de Trichoderma reesei ou d'une variante fonctionnelle de celle-ci, dans laquelle la variante fonctionnelle de l'hydrophobine provenant de Trichoderma reesei présente une identité de séquence d'acides aminés à la séquence signal de l'hydrophobine provenant de Trichoderma reesei d'au moins 80 %, en tant que signal de sécrétion, dans laquelle le peptide est capable de transmettre la sécrétion d'une protéine de fusion hétérologue à partir d'une cellule souche eucaryote.

2. Utilisation selon la revendication 1, dans laquelle l'hydrophobine est sélectionnée parmi HFBI et HFBII provenant de Trichoderma reesei.

3. Utilisation selon la revendication 1 ou 2, dans laquelle la séquence signal comprend au moins les dix acides aminés N-terminaux de la séquence d'acides aminés de HFBI ou au moins les quinze acides aminés N-terminaux de la séquence d'acides aminés de HFBII ou une variante fonctionnelle d'une de ces séquences avec une identité de séquence d'au moins 80 %.

4. Utilisation selon une des revendications 1 à 3, dans laquelle la séquence signal est sélectionnée parmi une des séquences des SEQ ID N° 1 à 14 ou une variante fonctionnelle de celles-ci avec une identité de séquence d'au moins 80 %.

5. Protéine de fusion hétérologue contenant un peptide tel que défini dans une des revendications 1 à 4.

6. Molécule d'acide nucléique codant pour une protéine de fusion selon la revendication 5.

7. Cassette d'expression, vecteur d'expression ou chromosome artificiel comprenant une molécule d'acide nucléique selon la revendication 6.

8. Utilisation d'une cassette d'expression, d'un vecteur d'expression et/ou d'un chromosome artificiel selon la revendication 7 pour la transformation ou transfection d'une cellule souche eucaryote.

9. Cellule souche eucaryote comprenant une cassette d'expression, un vecteur d'expression et/ou un chromosome artificiel selon la revendication 7.

10. Utilisation d'une cellule souche eucaryote selon la revendication 9 pour l'expression d'une protéine de fusion hétérologue selon la revendication 6.
